Europäisches Patentamt

European Patent Office

Office européen des brevets

(11) Numéro de publication: **0 376 770**
**A1**

(12) ## DEMANDE DE BREVET EUROPEEN

(21) Numéro de dépôt: **89403245.7**

(22) Date de dépôt: **23.11.89**

(51) Int. Cl.⁵: **A61K 39/395**, //C12N15/13

Le titre de l'invention a été modifié (Directives relatives à l'examen pratiqué à l'OEB, A-III, 7.3)

(30) Priorité: **23.11.88 FR 8815269**

(43) Date de publication de la demande:
**04.07.90 Bulletin 90/27**

(84) Etats contractants désignés:
**AT BE CH DE ES GB GR IT LI LU NL SE**

(71) Demandeur: **INSTITUT MERIEUX Société anonyme dite:**
**17, rue Bourgelat**
**F-69002 LYON(FR)**

(72) Inventeur: **Carosella, Edgardo D.**
**27, Rue Soeur Bouvier**
**F-69005 Lyon(FR)**
Inventeur: **Bach, Jean-François**
**180, rue de Grenelle**
**F-75007 Paris(FR)**
Inventeur: **Armand, Jacques Bernard**
**Lieudit le Clos Saint Germain sur l'Arbresle**
**F-69210 l'Arbresle(FR)**

(74) Mandataire: **Tonnellier, Jean-Claude et al**
**Cabinet Nony & Cie. 29, rue Cambacérès**
**F-75008 Paris(FR)**

(54) **Utilisation d'immunoglobulines G humaines, ou de fragments Fc de celles-ci, comme médicament préparatoire à l'administration chez l'homme d'anticorps monoclonaux.**

(57) Utilisation des fragments Fc d'IgG humaines dans la préparation d'un médicament préparatoire à l'administration chez l'homme, dans un but thérapeutique ou diagnostique, d'anticorps monoclonaux.

Le traitement préparatoire avec les fragments Fc permet de diminuer la fixation non spécifique et les effets secondaires des anticorps monoclonaux. Les doses d'anticorps monoclonaux peuvent être réduites.

EP 0 376 770 A1

**Utilisation de fragments Fc d'immunoglobulines G humaines, de peptides analogues du fragment Fc, ou d'immunoglobulines G humaines dans l'obtention d'un médicament préparatoire à l'administration chez l'homme, dans un but thérapeutique ou de diagnostic, d'anticorps monoclonaux.**

L'invention a pour objet l'utilisation de fragments Fc d'immunoglobulines G humaines (IgG), de peptides analogues du fragment Fc, capables d'être reconnus par les cellules portant un récepteur pour le fragment Fc des IgG, ou d'IgG humaines, dans la préparation d'un médicament préparatoire à l'administration d'anticorps monoclonaux, dans un but thérapeutique ou de diagnostic, chez l'homme.

L'utilisation de certains anticorps monoclonaux a été préconisée en thérapeutique, notamment dans le but d'éviter le rejet des greffes ou dans le traitement de certains cancers.

On sait que les lymphocytes T humains sont tous porteurs d'un antigène dit T3 et que certaines sous-populations de lymphocytes T portent des marqueurs tels que T4, T8, etc.... On sait également que les lymphocytes T activés comportent un site récepteur pour l'interleukine 2 (R-IL2). Les anticorps monoclonaux capables de reconnaître les antigènes T3, T4, T8 ou R-IL2 peuvent être administrés pour diminuer les populations de lymphocytes T, qui sont responsables du rejet des greffes d'organes. L'administration de tels anticorps monoclonaux est utilisée en thérapeutique pour éviter le phénomène de rejet des greffes (par exemple greffes de rein ou greffes de moëlle osseuse); voir notamment SOULILLOU et al, The Lancet, 13 June 1987, p.1339 New England Journal of Medicine, vol.313, p.337 (1985) article collectif; et Goldstein J., Transplantation, 1986, vol.42, p.507.

On a également préconisé l'utilisation d'anticorps monoclonaux, capables de réconnaître un antigène spécifique des cellules tumorales, et marqués, par exemple avec un marqueur radioactif, pour obtenir des images d'une tumeur, par exemple selon les méthodes de scintillographie; voir notamment Larson S.M., Journal of Nuclear Medicine, vol. 26, 538-545 (1985) et Schlom J. et al., in Cancer Principles and Practice of Oncology, vol.3, V.De Vita et al. Eds, Lippincott, Philadelphie (1985) pages 170 et seq.

D'une façon générale, les anticorps monoclonaux sont évidemment administrés dans un but de fixation spécifique sur une cible donnée.

Il est toutefois connu que le fragment Fc des IgG de souris, tout comme le fragment Fc des IgG humaines, est capable d'être reconnu par diverses cellules immunocompétentes portant à leur surface des récepteurs qui sont appelés "récepteurs pour le fragment Fc" (RFc). Ces récepteurs sont à l'origine d'une fixation (non spécifique de l'antigène) des immuno- globulines sur ces cellules, cette fixation non spécifique étant à l'origine de certains mécanismes de coopération avec lesdites cellules.

On sait en particulier que les IgG d'origine murine, y compris les IgG monoclonales, sont capables de se fixer aux récepteurs de haute et de basse affinité des cellules mononucléées humaines ; voir par exemple C.L. Anderson et al, Immunology Today, 7, 264 (1986). Par ailleurs, certains isotypes des immunoglobulines murines ont une activité biologique très importante et agissent notamment sur les cellules effectrices de la cytotoxicité anticorps-dépendante (ADCC), ainsi que sur les cellules NK (Natural Killer); voir notamment C.Anasetti et al., The Journal of Immunology, vol.138, 2979-2981 (1987).

Ces divers effets des IgG, et en particulier des anticorps monoclonaux, s'expliquent par leur fixation sur les récepteurs pour le fragment Fc de diverses cellules immunocompétentes.

C'est ainsi que des anticorps monoclonaux capables de reconnaître le marqueur T3 des lymphocytes T (par exemple les anticorps monoclonaux CD3 tels que les anticorps commerciaux OKT$_3$, qui sont des IgG2a de souris) induisent in vitro une réponse proliférative sur les cellules T humaines ; voir par exemple Van Wauwe J.P. et al. Journal of Immunology 124 2708 (1980) et Clement L.T. et al Journal of Immunology 135 165 (1985).

Ces interactions entre le fragment Fc des IgG murines et le récepteur pour le fragment Fc des cellules mononucléées humaines provoquent des effets secondaires indésirables ou une perte de l'activité spécifique des anticorps monoclonaux en thérapeutique humaine ainsi que dans les méthodes de diagnostic utilisant les techniques d'imagerie médicale. Cela conduit à augmenter les doses d'anticorps monoclonaux à administrer, avec des probabilités plus importantes d'apparition d'effets secondaires.

La présente invention a pour objet d'associer au traitement avec des anticorps monoclonaux, un traitement préparatoire avec des fragments Fc d'IgG humaines, ou encore avec des analogues desdits fragments Fc (peptides synthétiques, semi-synthétiques ou recombinants capables d'être reconnus par les cellules portant un récepteur pour le fragment Fc), ou avec des IgG humaines polyclonales, le but recherché étant l'occupation des RFc de façon à diminuer la fixation non spécifique des anticorps monoclonaux.

On sait que la reconnaissance spécifique de l'antigène par les IgG, y compris par les IgG monoclonales, ne constitue que la première étape de la destruction de l'antigène reconnu. L'étape ultérieure implique

2

généralement la fixation sur le fragment Fc d'une cellule immunocompétente ou d'un système lytique tel que le complément.

On pouvait donc redouter que l'occupation des sites récepteurs pour le fragment Fc des cellules immunocompétentes provoque l'inhibition des réactions ultérieures de destruction de l'antigène reconnu par les anticorps monoclonaux.

Or, on a maintenant découvert qu'il est possible, au contraire, grâce à l'administration préalable de fragments Fc d'IgG humaines, d'analogues du fragment Fc, ou d'IgG humaines de réduire les effets secondaires indésirables provoqués par l'administration d'anticorps monoclonaux, et d'éviter aussi la perte de l'activité spécifique de ces anticorps, sans compromettre la destruction de l'antigène. Cette méthode, qui rend plus efficace l'utilisation des anticorps monoclonaux, permet également de réduire les doses d'anticorps monoclonaux utilisées, et donc de diminuer encore les effets secondaires.

La présente invention a donc pour objet l'utilisation des fragments Fc des IgG humaines, des analogues du fragment Fc, ou d'IgG humaines,dans la préparation d'un médicament préparatoire à l'administration chez l'homme, dans un but thérapeutique ou de diagnostic, d'anticorps monoclonaux.

Dans le médicament selon l'invention, les IgG sont destinées à agir par fixation non spécifique sur les récepteurs pour le fragment Fc portés par les cellules immunocompétentes. Ces immunoglobulines n'étant pas utilisées dans un but de fixation spécifique, on comprend que les IgG utilisées sont des IgG polyclonales.

Les analogues du fragment Fc d'IgG humaines sont tous sous-fragments du fragment Fc, et plus généralement tous peptides synthétiques, semi-synthétiques ou recombinants comportant des séquences peptidiques présentes sur le fragment Fc et/ou susceptibles d'être reconnues par les récepteurs pour le fragment Fc des cellules mononucléées humaines. Parmi ces peptides, on peut citer ceux qui sont décrits dans les brevets US 4.752.601 et européen n° 0 227 110 ou dans la demande de brevet britannique n° 83.12679.

Pour préparer le médicament selon l'invention, on réalise par exemple des doses unitaires contenant de 200 à 1000 mg de fragments Fc, ou des doses molaires équivalentes d'IgG polyclonales humaines ou d'analogues du fragment Fc (peptides capables d'être reconnus par le RFc des cellules mononucléées humaines).

Le médicament selon l'invention se présente soit sous la forme de solutions injectables (solutions aqueuses isotoniques) soit sous la forme de préparations lyophilisées permettant de reconstituer des solutions injectables au moment de l'emploi.

Les IgG polyclonales sont obtenues, à partir de sang prélevé chez des donneurs, ou à partir de placentas, selon les méthodes classiques décrites dans la littérature ; voir par exemple Cohn E.J. et al., J. Am. Chem. Soc. 68, 459-475 (1946); Oncley J.L. et al., J. Am. Chem. Soc., 71, 541-550 (1949); P.Kistler et al., Vox Sang. 7, 414-424 (1962); et Taylor H.L. et al., J. Am. Chem. Soc. 78, 1356-1358 (1956). De préférence, on utilise des IgG modifiées ou intactes ayant subi un traitement propre à permettre leur administration par voie intraveineuse, par exemple selon les méthodes décrites par Sgouris J.T. Vox Sang. 13, 71-84 (1967), S.BARANDUN et al., Vox Sang. 7, 157-174 (1962), dans le brevet britannique 1.372.953 ou dans les brevets US 4.093.606 et 4.137.222.

Les fragments Fc peuvent être obtenus par clivage enzymatique des IgG humaines à l'aide de la plasmine ou de la papaïne, par exemple selon la méthode décrite par Barandun S. et al., Vox Sang. 28, 157 (1975). On sépare les fragments Fc des fragments Fab ou des IgG intactes selon les techniques connues, par exemple par filtration sur gel.

Comme indiqué ci-dessus, le médicament à base de fragments Fc ou analogues, ou d'IgG, est utilisé dans un traitement préparatoire à l'administration d'anticorps monoclonaux, en particulier d'anticorps monoclonaux dirigés contre les cellules T ou contre les cellules tumorales.

On sait que les fragments Fc d'IgG humaines, et dans une certaine mesure les IgG polyclonales humaines, ont un effet favorable sur l'inhibition du rejet des greffes d'organes ; voir par exemple le brevet européen 0 147 283. Cet effet propre s'ajoutera à celui des anticorps monoclonaux anti-T.

Les anticorps spécifiques des cellules T sont notamment les anticorps capables de reconnaître le marqueur T3, présent sur toutes les cellules T, ou le marqueur T4 ou T8 qui est présent sur certaines cellules T. On peut utiliser en particulier les anticorps monoclonaux anti- T4 ou anti-T8, les anticorps YT35 capables de reconnaître une glycoprotéine de surface présente sur les cellules T, mais non sur les cellules B, ou encore les anticorps anti-TAC (TAC : récepteur pour l'interleukine-2), ces derniers anticorps reconnaissant spécifiquement les lymphoblastes T (lymphocytes T activés).

Les anticorps monoclonaux dirigés contre les cellules T sont disponibles dans le commerce. Ils sont commercialisés notamment par les firmes Ortho et Becton-Dickinson.

Les anticorps monoclonaux antitumoraux sont utilisés soit en thérapeutique antitumorale, soit en imagerie médicale dans les techniques scintillographiques ou analogues.

Parmi les anticorps monoclonaux antitumoraux, marqués ou non, disponibles dans le commerce, on citera par exemple les anticorps anti-CALLA (human Cancer Acute Lymphoblastic Leukemia Antigen), commercialisé par Becton-Dickinson.

L'invention a également pour objet une méthode de traitement préparatoire à l'administration d'anticorps monoclonaux chez l'homme, caractérisée par le fait que l'on administre antérieurement une préparation contenant des fragments Fc d'IgG humaines, ou des analogues du fragment Fc, tels que définis précédemment, ou encore des IgG humaines polyclonales.

Le traitement préparatoire est effectué par exemple de 30 minutes à 1 heure avant chaque administration d'anticorps monoclonaux.

On administre généralement les fragments Fc par voie intraveineuse à raison de 30 à 80 mg par kg de poids corporel et par jour. Pour les IgG intactes ou les peptides analogues du fragment Fc, on utilise des doses molaires équivalentes à celles qui viennent d'être indiquées pour le fragment Fc.

L'invention a également pour objet un procédé de préparation d'un médicament préparatoire à l'administration, chez l'homme, d'anticorps monoclonaux. Ce procédé consiste par exemple à dissoudre l'ingrédient actif tel que défini ci-dessus, dans le cas où il est sous forme lyophilisée, dans un véhicule pharmaceutique aqueux compatible avec l'administration parentérale, et/ou à réaliser avec l'ingrédient actif en solution dans un tel véhicule pharmaceutique aqueux des doses unitaires telles que définies ci-dessus.

Les exemples suivants illustrent l'invention sans toutefois la limiter.

## EXEMPLE 1- ESSAIS IN VITRO

### 1. Préparation des lymphocytes du sang périphérique

Du sang veineux hépariné a été collecté chez des donneurs sains volontaires des deux sexes, âgés de 25 à 45 ans et dilué avec un volume de NaCl 0,15 M. Les cellules mononucléées (CMN) du sang périphérique de chaque donneur ont été isolées individuellement par centrifugation sur gradient de Ficoll-Radioselectan (concentration finale : 6,3/9,9%, densité 1,077), selon la technique de Boyüm A., Scand.J. Clin. Lab. Invest. 21 (suppl. 97), 77, 1968. Les CMN de l'interface ont été collectées et lavées une fois avec du milieu RPMI 1640 (GIBCO 041-01875) additionné de 10% de sérum de veau foetal (SVF; origine : Flow). Les cellules ont été utilisées immédiatement ou congelées et conservées dans de l'azote liquide.

### 2. Fragments Fc d'IgG humaines

Les fragments Fc sont préparés à partir d'IgG humaines purifiées par la technique d'Oncley J.L. et al, J.Am. Chem. Soc. 71, 541, 1949, et traitées par la plasmine humaine selon la technique de Barandum S., Vox. Sang. 28, 157, 1975. Les IgG proviennent soit de sang prélevé à la veine soit de sang exprimé à partir de placentas.

La solution de fragments Fc est purifiée par chromatographie de filtration sur gel de ACA44 (Pharmacia). Sa pureté est de l'ordre de 96%.

Elle contient 1,5% de fractions 7S et 2,5% d'un pic intermédiaire. Les fragments Fab contiennent au plus 2% de fragments Fc.

On décrira ci-après les résultats obtenus avec trois lots différents de fragments Fc :

| Lot n° | Origine |
|--------|---------|
| 001 | placentaire |
| 002 | plasmatique |
| 003 | plasmatique |

### 3. Fixation du fragment Fc à la protéine A

a) Absorption des fragments Fc, des fragments Fab et des IgG dans des cupules, et saturation à l'aide d'albumine.

Chaque lot de fragments Fc, de fragments Fab ou d'IgG a été dilué à 100µg/ml en tampon carbonate et réparti à raison de 100µl par cupule. Après une incubation d'une nuit à + 4°C, les plaques ont été rincées plusieurs fois avec un tampon PBS-Tween.

b)Saturation des cupules

Après rinçage, 250µl de solution saline physiologique à 5% de BSA (sérum albumine bovine) ont été ajoutés à chaque cupule et incubés pendant 1 heure à 37°C.

c) Test RIA

100µl de protéine A de staphylocoque, marquée à l'iode 125, à différentes concentrations : 1/50, 1/250, 1/1250 ont été répartis dans chaque cupule et incubés pendant 2 heures à 37°C. Après incubation, on effectue trois lavages avec un tampon PBS-Tween.

d) Résultats du test RIA

Les résultats, exprimés en cpm (coups par minute) sont résumés dans le Tableau 1 ci-après.

Ce test montre que le fragment Fc a conservé la capacité de se lier à la protéine A (comme les IgG intactes), alors que les fragments Fab ne se fixent pas.

## 4.Fragment Fc et reconnaissance des récepteurs Fc gamma des cellules mononucléées humaines

a) Préparation des complexes Erythrocytes-Anticorps (complexes EA)

Les complexes sont constitués d'érythrocytes de boeuf (E) et d'anticorps (IgG) de lapin anti-globules rouges de boeuf (A). Les globules rouges de boeuf (GRB) (origine : Rhône Mérieux, France) sont conservés 3 semaines au maximum. Au moment de l'emploi, ils sont lavés plusieurs fois avec une solution saline physiologique et centrifugés. On réalise une suspension à 5% du culot de centrifugation en tampon PBS ; on mélange alors 1ml de cette suspension avec 1 ml de l'anticorps dilué au 1/50 (1ère dilution non agglutinante). L'ensemble est incubé 30 minutes à 37°C. A la fin de l'incubation, le mélange est lavé deux fois avec du milieu RPMI 1640 et centrifugé, et le culot est repris pour préparer une suspension finale à 0,5% en RPMI 1640.

b) Formation des rosettes EA

On mélange 100µl d'une suspension lymphocytaire (obtenue comme en 1) ci-dessus) à 5x10⁶ cellules/ml en solution de Hanks et 100µl de complexe EA. Après centrifugation pendant 5 minutes à 800 rpm, à + 4°C, le mélange est incubé à +4°C pendant 2 heures. Les cellules sont mises en suspension très doucement. La lecture se fait sur cellules de numération (chambre de comptage). Une rosette est définie par la présence d'au moins 3 globules rouges de boeuf autour d'un lymphocyte. Il faut compter 400 cellules (lymphocytes) dans différentes zones de la chambre de comptage.

c) Inhibition de la formation des rosettes EA par le fragment Fc, Fab ou les IgG

Différentes concentrations de fragments Fc, de fragments Fab ou d'IgG sont préparées extemporanément. On mélange successivement 0,1 ml de lymphocytes à une concentration de 5x10⁶cellules/ml avec 0,1 ml de différentes dilutions des produits à tester pendant 30 minutes à 37°C. Après centrifugation

5

pendant 10 minutes à 800 rpm, et aspiration du surnageant, le culot est repris dans 100µl de milieu RPMI et incubé avec 100µl de complexe EA à 0,5% pendant 1 heure à +4°C. Les étapes successives sont les mêmes qu'en b).

Les résultats sont résumés dans le Tableau 2 ci-après, sous la forme du pourcentage de rosettes EA observé.

Ces résultats montrent qu'après incubation des cellules mononuclées du sang périphérique avec le fragment Fc des IgG, il y a une inhibition de la formation de rosettes EA. Cette inhibition augmente en proportion directe avec la quantité de produit introduit.

On sait que la formation de rosettes passe par la fixation des anticorps anti-érythrocytes, par leur fragment Fc, sur des cellules T portant des RFc. On peut donc conclure que les fragments Fc testés ont conservé leur capacité de fixation à ces récepteurs.

## 5. Cytotoxicité anticorps dépendante à médiation cellulaire (ADCC)

Le test est effectué selon la méthode de F. WISLOFF et al., Scand. J. Immunol., 3, 29-38 (1974).

### a) Préparation des cellules cibles

Globules rouges de poulet (GRP) : Les GRP, après des lavages successifs en tampon PBS, ont été ajustés à une concentration de 100x10⁶cellules/ml RPMI à 10% SVF (le sérum de veau foetal a été préalablement soumis à une absorption sur des GRP). Une incubation d'une heure à 37°C est effectuée avec 100µl de GRP en présence de 100µl de sérum de lapin anti GRP au 1/1000 (Cappel) et 100µl de Chrome 51 (activité = 100 mCi/mg Cr).

Parallèlement, un tube témoin est préparé dans les mêmes conditions, en remplaçant le sérum de lapin anti-GRP par du sérum de lapin normal (1/1000). Après deux lavages en RPMI, la suspension de GRP sensibilisés et marqués a été ajustée à 200.000 cellules/ml en RPMI à 10% SVF.

### b) Test ADCC

Les cellules effectrices (lymphocytes humains de sang périphérique obtenus selon 1. ci-dessus) ont été dénombrées et ajustées à une concentration de 5x10⁶/ml en RPMI à 10% SVF.

A l'aide d'une seringue Hamilton, on a distribué dans des microplaques à fond rond (NUNC Delta) 100µl de cellules effectrices et 50µl des produits à tester, à différentes dilutions, chaque dilution faisant l'objet de trois essais. Après une incubation de 20 min. à 37°C, 50µl de GRP sensibilisés et marqués ont été distribués, donnant un rapport final de 50 cellules effectrices pour 1 cellule cible (50:1).

Le relargage spontané de chrome a été déterminé en incubant les GRP avec du milieu RPMI à 10% SVF, le relargage maximum étant obtenu en ajoutant aux GRP de l'acide chlorhydrique 1N. Comme témoin de l'activité de l'antisérum, les cellules effectrices ont été mises en présence de GRP préincubés avec du sérum de lapin normal.

Les plaques, après une incubation pendant 18h à 37°C sous atmosphère humide à 5% CO₂, ont été centrifugées pendant 10 minutes à 800rpm et la récupération du surnageant a été effectuée sur des tampons filtres (Flow, Ref. 78-21-005).

### c) Résultats

Les résultats, exprimés en pourcentage de cytotoxicité sont résumés dans le Tableau 3 ci-après.

On observe que le fragment Fc d'origine placentaire ou plasmatique inhibe, de façon proportionnelle à la dose introduite, la cytotoxicité dependant des anticorps. L'effet inhibiteur est supérieur à celui induit par les IgG à la même dose. L'effet inhibiteur est absent avec les fragments Fab.

On sait que l'effet cytotoxique anticorps-dependant implique la fixation des complexes cellule cible-anticorps sur les cellules K, qui possèdent des récepteurs pour le fragment Fc des IgG. On peut donc conclure que les fragments Fc testés ont conservé cette propriété d'être reconnus par les RFc des cellules K.

**6. Test de prolifération des CMN : inhibition de la réponse proliférative induite par l'anticorps monoclonal OKT3.**

a) Les cellules (CMN), ajustées à $1.10^6$ cellules/ml en milieu RPMI 1640 supplémenté par 10% de SVF, sont distribuées dans les puits d'une plaque (NUNC - Fond plat - 96 puits) à l'aide d'une seringue Hamilton.

Chaque essai est réalisé dans trois puits identiques à raison de :

- 100μl de la suspension cellulaire (100 000 cellules/puits),
- 50μl de milieu RPMI + 10% SVF ou de produit à tester convenablement dilué (Fc, Fab ou IgG à 400 ; 200 ; 100 ; 10 ; 1 0,1 μg/ml),
- 50μl de mitogène : phytohémagglutinine (PHA : Wellcome, 1/400e) ou anticorps monoclonal à étudier : OKT3 (IgG2a, origine : Ortho- Diagnostics), dose optimale de 0,1 ng/ml. Les plaques sont alors conservées à l'étuve à 37°C, sous atmosphère humidifiée (95% air - 5% $CO_2$) pendant 48 heures. 50μl d'une solution de Thymidine tritiée diluée au 1/50e dans du milieu RPMI sont ajoutés dans chaque puits (2μCi/puits). Après 18 h d'incubation à 37°C, les plaques sont placées à +4°C pour arrêter l'incorporation de $^3H$ par les cellules en culture.

Les cellules sont recueillies sur des filtres en fibre de verre (Whatman) à l'aide d'un appareil de récolte de cellules (harvester) de type Skatron. Chaque filtre, préalablement séché puis découpé, est placé dans un tube contenant 3 ml d'une solution scintillante (Lipofluor).

Le taux de radioactivité est évalué par un compteur de radioactivité bêta. Les résultats sont exprimés en coups par minute (cpm), la moyenne et l'écart-type sont calculés à partir des valeurs obtenues pour 8 individus.

b) Résultats

Les résultats (taux de prolifération cellulaire) sont résumés dans les Tableaux 4 à 6 ci-après.

Les essais effectués montrent que le fragment Fc des IgG n'induit pas de prolifération lymphocytaire dans les conditions de culture décrites précédemment (Tableau 4). Le fragment Fc est capable d'inhiber la réponse proliférative induite par les anticorps monoclonaux OKT3. Cette inhibition est proportionnelle à la concentration du fragment Fc introduit dans le test (Tableaux 5 et 6). Les IgG ont aussi cette capacité mais à un degré moins important.

Conclusion de l'exemple 1

Les essais in vitro rapportés ci-dessus avaient pour objectif l'exploration des propriétés physicochimiques du fragment Fc et l'étude de sa capacité à se fixer aux récepteurs pour le fragment Fc.

Le premier essai montre que le fragment Fc des IgG humaines est capable de se fixer à la protéine A, ce qui permet de conclure que son site de fixation situé sur la région CH2 et CH3 est conservé ; CICCIMARRA F. et al, PNAS, 72 56) 2081-2083, 1975. Les autres essais montrent que le fragment Fc est aussi capable de se fixer aux récepteurs RFc, ce qui confirme l'intégrité des structures CH2 et CH3; voir Sarmay G., Europ. Journal of Immunology, vol.15, 1037 (1985).

La possibilité d'inhiber la réponse proliférative induite par l'anticorps monoclonal OKT3 montre que le récepteur Fc monocytaire capable de fixer le fragment Fc des IgG humaines est le même que celui qui fixe les IgG2a de souris.

EXEMPLE 2

ESSAI THERAPEUTIQUE

Effet du fragment Fc des IgG en association avec l'anticorps monoclonal OKT3 dans le traitement prophylactique du rejet de greffe de rein.

a) Protocole de traitement de receveurs d'allogreffe rénale par le fragment Fc d'IgG humaines (lot 003) et des anticorps monoclonaux (OKT3).

Trois patients receveurs d'allogreffe rénale et traités par l'anticorps monoclonal OKT3 (dose 5mg/jour; voie intraveineuse) ont reçu une injection de fragments Fc humains avant les 5 premières injections d'anticorps monoclonal. Les fragments Fc sont administrés à raison de 50mg/kg/jour pendant 5 jours, en perfusion lente 1 heure avant l'injection de l'anticorps OKT3.

Le but de cet essai thérapeutique était notamment d'observer une éventuelle diminution de l'intensité des effets secondaires associant entre autres fièvre, diarrhée, frissons, syndrome méningé, que l'on observe chez tous les patients après les trois premières injections d'OKT$_3$.

b) Résultats

Les résultats obtenus ont montré que chez les trois patients ayant reçu des fragments Fc, l'intensité du syndrome clinique est diminuée. Par ailleurs, la fièvre qui, chez les patients traités par les anticorps OKT3, débute invariablement 12 heures après la première injection, n'apparaît qu'après la troisième injection chez les patients traités par les fragments Fc.

Le suivi biologique des patients a montré que la déplétion massive en cellules T périphériques induite par l'OKT3 n'est pas modifiée par l'injection de fragments Fc. Cela montre que la fixation des fragments Fc sur les RFc n'empêche pas la destruction de l'antigène (ici les cellules T), contrairement à ce qu'on pouvait redouter.

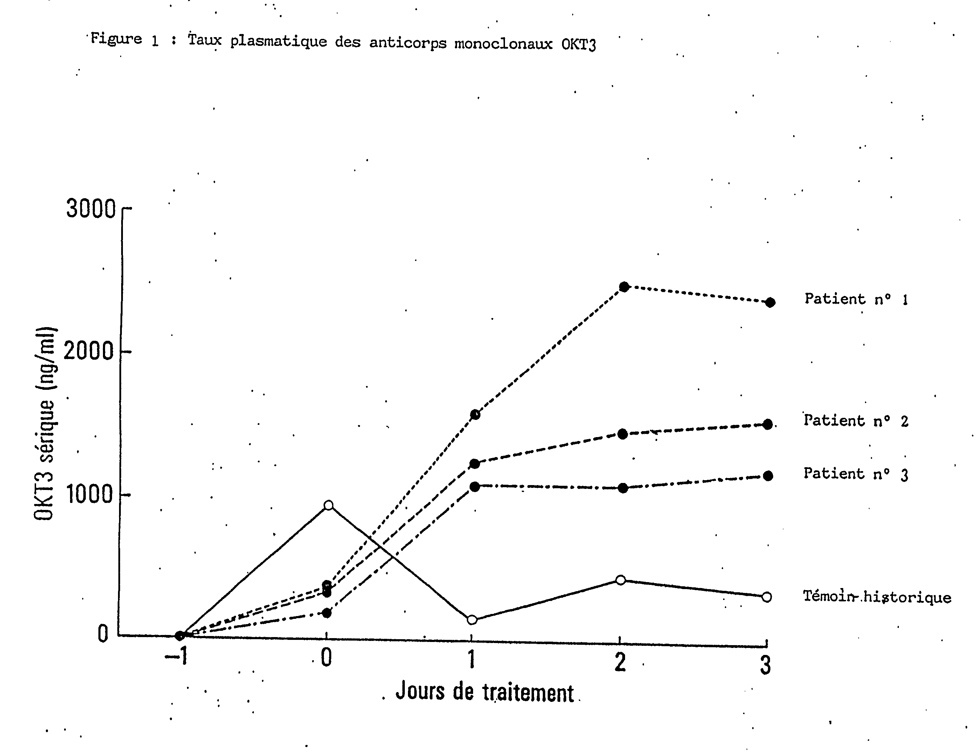

Par ailleurs, les dosages d'anticorps OKT3 circulants (par test ELISA) montrent (par rapport à un témoin historique), que, chez les trois patients ayant reçu des fragments Fc, les taux d'OKT3 circulants atteignent un plateau (voir figure 1). Cela plaide en faveur d'une biodisponibilité accrue du produit, rendue possible par l'injection des fragments Fc.

Les essais cliniques confirment donc les résultats des expériences réalisées in vitro. Ces essais permettent de conclure que l'association thérapeutique du fragment Fc des IgG, en administration préalable à celle des anticorps monoclonaux, augmente l'efficacité thérapeutique et diminue les effets secondaires. On peut donc envisager de réduire les doses d'anticorps monoclonaux.

## Tableau 1 : Fixation*de la protéine A

| Conc.<br>Lot n° | 25 µg/ml | 50 µg/ml | 100 µg/ml | 200 µg/ml |
|---|---|---|---|---|
| FC 001 | 78 621 | 86 872 | 97 616 | 107 711 |
| FC 002 | 81 071 | 70 062 | 83 749 | 134 847 |
| FC 003 | 99 687 | 92 227 | 100 015 | 101 256 |
| Témoin Fab | 1 996 | 1 463 | 974 | 1 110 |
| Témoin IgG | 202 485 | 216 286 | 222 362 | 247 106 |

* cpm

Témoin Blanc : 567

Tableau 2

| Pourcentage de Rosettes EA * | | | | | | | | |
|---|---|---|---|---|---|---|---|---|
| Lot n° | Témoin (RPMI) | Concentration en fragment Fc ( μg/ml) | | | | | | |
| | | 1 | 5 | 10 | 20 | 50 | 100 | 200 |
| [a] 001 | 13,7±2,9 | 10,2±3,5 | 9,3±3,1 | 8,2±3,2 | 7,2±3,1 | 5,9±2,8 | 4,6±2,3 | 3,1±2,2 |
| [b] 002 | 15,2±4,3 | 12,2±3,3 | 12,2±4,8 | 10,2±4,5 | 9,5±2,6 | 8,5±2,6 | 7,5±1,9 | 6 ±2,6 |
| [b] 003 | 15,2±4,3 | 11,7±5,5 | 12,2±4,4 | 10,2±3,3 | 10 ±2,9 | 8,2±1,5 | 8,7±2,1 | 6,2±1,5 |

a : n = 14

b : n = 4

n = nombre d'individus testés

* : moyenne ± écart-type

Tableau 3

| Pourcentage de cytotoxicité*(test ADCC) | | | | |
|---|---|---|---|---|
| Produit | Concentration μg/ml | | | |
| | 0 | 6 | 12 | 14 |
| Fc lot 001[**] | 47,4 ± 7,4 | 25,2 ± 5,5 | 19,1 ± 4,56 | 15,5 ± 3,5 |
| Fc lot 002[***] | 43,2 ± 6,1 | 19 ± 2,6 | 17,8 ± 2,2 | 15,6 ± 2,1 |
| Fc lot 003[***] | 43,2 ± 6,1 | 18,3 ± 3,1 | 16 ± 3,3 | 14 ± 2,9 |
| Témoin Fab[**] | 47,4 ± 7,4 | 46,8 ± 8,2 | 47,9 ± 7,6 | 46,4 ± 7 |
| Témoin IgG[**] | 47,4 ± 7,4 | 33,1 ± 5,8 | 30,6 ± 5,5 | 28,0 ± 5,7 |

* % cytotoxicité : Relarg.Cr51 (moyenne) ± écart type

[**] n = 6

[***] n = 4

n = nombre d'individus testés

Tableau 4

| Taux de prolifération cellulaire*obtenus après culture des CMN en présence de fragments Fc ou Fab ou d'IgG | | | |
|---|---|---|---|
| Doses µg/ml | Produits | | |
| | Fc lot n° 001 | Témoin Fab | Témoin IgG |
| 400 | 1966 ± 260,5 | 2545,9 ± 870,9 | 1575,4 ± 117,3 |
| 200 | 1719,0 ± 221,6 | 2250,4 ± 449,6 | 1730,4 ± 207,6 |
| 100 | 1975,0 ± 266,3 | 2209,2 ± 526,3 | 2136,1 ± 283,2 |
| 10 | 1753,6 ± 253,1 | 2209,7 ± 599,9 | 2234,4 ± 306,9 |
| 1 | 1627,7 ± 202,8 | 3124,7 ± 829,0 | 1739,4 ± 268,2 |
| 0,1 | 1749,6 ± 259,2 | 3887,0 ± 848,6 | 1684,1 ± 215,2 |
| 0,01 | 1572,5 ± 281,8 | 1339,7 ± 288,3 | 1974,1 ± 368,4 |
| 0 | 1960,9 ± 334,1 | 1960,9 ± 334,1 | 1960,9 ± 334,1 |
| Comparaison = PHA (1/400e):45190,7 + 3270,9 | | | |

* cpm (moyenne) ± erreur standard
nombre d'individus testés : n = 8

Tableau 5

| Comparaison du taux de prolifération*des cellules du sang périphérique après stimulation par l'OKT3 en présence du fragment Fc ou Fab ou des IgG | | | |
|---|---|---|---|
| Doses µg/ml | Essais | | |
| | Fc lot 001 | Fab | IgG |
| 400 | 1790,9 ± 381,9 | 60791,2 ± 4164,8 | 7867,9 ± 990,8 |
| 200 | 1779,1 ± 153,6 | 72795,6 ± 3798,7 | 17935,0 ± 2274,7 |
| 100 | 2395,9 ± 417,1 | 76745,5 ± 3739,9 | 24162,4 ± 3043,9 |
| 10 | 3666,6 ± 599,1 | 76082,0 ± 3419,5 | 62239,9 ± 3475,9 |
| 1 | 23099,9 ± 2459,3 | 79809,4 ± 6014,7 | 72001,6 ± 3729,6 |
| 0,1 | 39800,6 ± 3134,9 | 79154,1 ± 4177,3 | 73188,4 ± 4057,9 |
| 0,01 | 52670,6 ± 3866,6 | 88430,9 ± 4456,9 | 77619,7 ± 5288,7 |

* cpm (moyenne) ± erreur standard
n = 8
Comparaison : RPMI : 1960,9 ± 334,1
OKT3 (0,1 ng/ml) : 80463,6 ± 4201,6
PHA (1/400e) : 45190 ± 3270,9

Tableau 6

| Inhibition par Fc de la réponse proliférative induite par l'anticorps monoclonal OKT3 | | | | | | | | | |
|---|---|---|---|---|---|---|---|---|---|
| Donneurs | D.O. | R.D. | A.P. | R.C. | L.B. | D.B. | C.R. | A.F. | cpm** |
| RPMI* | 1175 | 861 | 854 | 331 | 979 | 1085 | 1574 | 322 | 897 ± 419 |
| PHA* | 66512 | 30643 | 57519 | 54214 | 73444 | 42169 | 59687 | 22563 | 50843 ± 17621 |
| OKT3* 0,1 ng/ml | 69880 | 75324 | 92077 | 85616 | 94388 | 64779 | 74918 | 47928 | 75613 ± 15310 |
| lot 002 | | | | | | | | | |
| 0,1 µg/ml | 33385 | 61976 | 62533 | 55325 | 43818 | 53350 | 50355 | 24203 | 48118 ± 13583 |
| 1 µg/ml | 18388 | 38726 | 44066 | 47891 | 37544 | 41725 | 21931 | 11287 | 32694 ± 13525 |
| 10 µg/ml | 1967 | 5351 | 4364 | 6643 | 2968 | 6583 | 2957 | 611 | 3930 ± 2182 |
| lot 003 | | | | | | | | | |
| 0,1 µg/ml | 76074 | 76464 | 66474 | 83903 | 81047 | 59814 | 70625 | 33195 | 68199 ± 16119 |
| 1 µg/ml | 38916 | 54848 | 55356 | 72846 | 62529 | 55935 | 40052 | 16819 | 49662 ± 17271 |
| 10 µg/ml | 7975 | 13839 | 10473 | 15102 | 8475 | 11515 | 4449 | 660 | 9061 ± 4791 |

* Comparaison

** moyenne ± écart-type

## Revendications

1. Utilisation des fragments Fc d'IgG humaines, ou des analogues du fragment Fc, ou d'IgG polyclonales humaines, dans la préparation d'un médicament préparatoire à l'administration chez l'homme, dans un but thérapeutique ou diagnostique, d'anticorps monoclonaux.

2. Utilisation selon la revendication 1, caractérisée par le fait que ledit médicament est destiné à être administré de 30 minutes à 1 heure avant chaque administration d'anticorps monoclonaux.

3. Utilisation selon l'une quelconque des revendications précédentes, caractérisée par le fait que lesdits analogues du fragment Fc sont des peptides synthétiques, semi-synthétiques ou recombinants comportant des séquences peptidiques présentes sur le fragment Fc et/ou susceptibles d'être reconnues par les récepteurs pour le fragment Fc des cellules mononucléées humaines.

4. Utilisation selon l'une quelconque des revendications précédentes, caractérisée par le fait que ledit médicament est destiné à être administré à raison de 30 à 80 mg de fragments Fc par kg de poids corporel et par jour, ou à des doses molaires équivalentes d'IgG polyclonales humaines ou d'analogues du fragment Fc.

Figure 1 : Taux plasmatique des anticorps monoclonaux OKT3

EP 0 376 770 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | WO-A-8 802 594 (NEORX CORP.) <br> * En entier * <br> --- | 1-4 | A 61 K 39/395// <br> C 12 N 15/13 |
| X | WO-A-8 704 351 (THE GENERAL HOSPITAL CORP.) <br> * Page 11, ligne 5 - page 13, ligne 12; exemples 5,6,11; revendications * <br> --- | 1-4 | |
| X | JOURNAL OF NUCLEAR MEDICINE, vol. 29, no. 1, janvier 1988, page 39; J.A. CARRASQUILLO et al.: "Effect of Antibody Dose on the Imaging and Biodistribution of Indium-111 9.2.27 Anti-Melanoma Monoclonal Antibody" <br> * Page 42, colonne 2, lignes 24-29; page 45, colonne 1, lignes 33-41 * <br> --- | 1-4 | |
| A | EP-A-0 227 110 (TEIJIN LTD) <br> * Page 2 * <br> --- | 3 | |
| D,A | EP-A-0 147 283 (INSTITUT MERIEUX) <br> ----- | | DOMAINES TECHNIQUES RECHERCHES (Int. Cl.5) <br><br> A 61 K <br> C 12 N |

Le présent rapport a été établi pour toutes les revendications

| Lieu de la recherche | Date d'achèvement de la recherche | Examinateur |
|---|---|---|
| LA HAYE | 28-02-1990 | SKELLY J.M. |